Europäisches Patentamt

European Patent Office

Office européen des brevets

(11) Numéro de publication: **0 335 804**
**A1**

(12) ## DEMANDE DE BREVET EUROPEEN

(21) Numéro de dépôt: **89400899.4**

(22) Date de dépôt: **31.03.89**

(51) Int. Cl.⁴: **A 61 K 39/395**
**// C12N5/00**

(30) Priorité: **01.04.88 FR 8804402**

(43) Date de publication de la demande:
**04.10.89 Bulletin 89/40**

(84) Etats contractants désignés:
**AT BE CH DE ES GB GR IT LI LU NL SE**

(71) Demandeur: **INSTITUT MERIEUX Société anonyme dite:**
**17, rue Bourgelat**
**F-69002 LYON (FR)**

(72) Inventeur: **Bonneville, Marc**
**6, Allée des Coucous**
**F-44000 Nantes (FR)**

**Carcagne, Jean**
**Impasse Marcel Mérieux**
**F-69280 Marcy l'Etoile (FR)**

**Carosella, Edgardo D.**
**27, Rue Soeur Bouvier**
**F-69005 Lyon (FR)**

**Latour, Mireille**
**23, Lotissement des Sabines**
**Chemin de Granvaud F-69130 Ecully (FR)**

**Moreau, Jean-François**
**3, Impasse des Bois**
**F-44120 Vertou (FR)**

**Soulillou, Jean-Paul**
**32 ter, Rue de l'Abbaye**
**F-44100 Nantes (FR)**

(74) Mandataire: **Nony, Michel et al**
**Cabinet NONY & CIE 29, rue Cambacérès**
**F-75008 Paris (FR)**

(54) **Procédé de préparation d'un sérum antilymphocytaire par immunisation d'animaux à l'aide d'un clone de lymphoblaste T humain, et sérum antilymphocytaire ainsi obtenu.**

(57) Procédé de préparation d'un sérum antilymphocytaire, dans lequel on immunise des animaux vertébrés à l'aide d'un immunogène constitué par des cellules lymphoïdes humaines, et que l'on recueille au bout d'un temps suffisant les anticorps sériques des animaux immunisés, caractérisé par le fait que l'on prépare un clone de lymphoblaste T humain sensibilisé contre des cellules allogéniques, et que l'on utilise des cellules de ce clone comme immunogène et sérum antilymphocytaire obtenu par ce procédé.

EP 0 335 804 A1

## Description

**Procédé de préparation d'un sérum antilymphocytaire par immunisation d'animaux à l'aide d'un clone de lymphoblaste T humain, et sérum antilymphocytaire ainsi obtenu.**

La présente invention a pour objet un procédé de préparation d'un sérum antilymphocytaire par immunisation d'animaux à l'aide d'un clone de lymphoblaste T humain, le sérum antilymphocytaire ainsi obtenu et son utilisation comme médicament.

On sait que le rejet des allogreffes fait intervenir essentiellement les lymphocytes T dits activés qui, par contact avec les antigènes cellulaires du donneur, se sont transformés en lymphoblastes cytotoxiques qui détruisent les cellules du greffon.

On a déjà proposé de lutter contre l'effet cytotoxique des cellules T du porteur de greffe par administration de sérum antilymphocytaire (SAL). Les SAL sont des anticorps sériques obtenus par immunisation d'animaux, tels que des lapins ou des chevaux, à l'aide de lymphocytes xénogéniques, en particulier de lymphocytes ou de thymocytes humains dans le cas de la préparation de SAL destinés aux humains. Le sérum des animaux immunisés contient notamment des anticorps dirigés contre les lymphocytes T humains, et de tels sérums, dits antilymphocytaires, ont un effet immuno-suppresseur qui a été démontré chez l'homme, et sont utilisés dans la prévention du rejet des greffes.

Toutefois, l'utilisation des SAL chez l'homme présente des inconvénients en raison d'effets secondaires indésirables. En effet, l'agent immunogène est constitué de lymphocytes, et il est très difficile d'éviter la présence de divers contaminants cellulaires, notamment polynucléaires, monocytes, globules rouges et plaquettes. Il en résulte la production, par les animaux immunisés, d'anticorps contaminants correspondants qu'il est difficile d'éliminer complètement et qui sont à l'origine, de complications fréquentes, notamment leucopénie, hypoplaquettose, anémie hémolytique, etc...

On a déjà proposé diverses méthodes de purification des SAL, dans le but d'éliminer certains anticorps contaminants. Toutefois ces méthodes sont généralement fastidieuses, et il est difficile d'obtenir une purification poussée sans perte d'une part importante de l'activité immuno-suppressive.

La présente invention a pour objet de remédier à ces inconvénients en proposant l'utilisation comme immunogène, exempt de contaminants cellulaires, d'un clone de lymphoblaste T humain. Autrement dit, il s'agit d'une cellule T ayant déjà été sensibilisée contre des cellules allogéniques, et ayant ainsi subi la transformation lymphoblastique. L'utilisation comme agent immunogène d'un clone de lymphoblaste permet d'obtenir la production d'anticorps plus spécifiques des lymphocytes, et en particulier des lymphocytes activés (lymphoblastes), qui sont impliqués dans le rejet du greffon. Un autre avantage du clone cellulaire est son homogénéité qui garantit une bonne constance des propriétés du sérum antilymphocytaire obtenu, alors qu'avec les SAL classiques, les variations d'un lot de fabrication à un autre peuvent être importantes.

On a découvert en outre que, de façon surprenante, les SAL obtenus selon la présente invention, bien que constitués essentiellement d'anticorps dirigés contre une cellule unique, ont le même caractère polyvalent que les SAL obtenus selon les méthodes classiques.

On notera enfin que le sérum antilymphocytaire de l'invention évite l'utilisation chez l'homme d'anticorps produits par des lignées cancéreuses (anticorps monoclonaux) dont les effets à long terme sont actuellement inconnus, et qui risquent de favoriser la formation, chez les personnes traitées, d'anticorps anti-idiotypiques qui inhibent l'action des anticorps administrés.

La présente invention a donc pour objet un procédé de préparation d'un sérum antilymphocytaire, dans lequel on immunise des animaux vertébrés à l'aide d'un immunogène constitué par des cellules lymphoïdes humaines, et l'on recueille au bout d'un temps suffisant les anticorps sérique des animaux immunisés, caractérisé par le fait que l'on prépare un clone de lymphoblaste T humain sensibilisé contre des cellules allogéniques, et que l'on utilise des cellules de ce clone comme immunogène.

Selon un mode de réalisation particulier, pour préparer ledit clone, on sensibilise des lymphocytes T humains à l'aide de cellules allogéniques, puis l'on isole, selon les méthodes connues, un clone de lymphoblaste allosensibilisé. En outre, on peut cultiver le clone par stimulations successives à l'aide de cellules, en particulier de lymphocytes B, allogéniques. Il est particulièrement avantageux d'utiliser comme source de cellules stimulantes des lignées de cellules allogéniques, par exemple des cellules B, transformées de façon à être cultivables in vitro. Il est possible, mais non nécessaire, de procéder au clonage de ces lignées. Ces lignées peuvent être obtenues par exemple par transformation desdites cellules allogéniques, selon les méthodes connues, avec un virus tel que le virus d'Epstein-Barr. Selon un mode de réalisation particulier, les cellules stimulantes, transformées ou non, proviennent du même individu que celles ayant servi à réaliser l'allosensibilisation. On peut toutefois utiliser comme cellule stimulante toute cellule portant la spécificité HLA-D reconnue par le clone de lymphoblaste T considéré.

La réaction d'allosensibilisation peut être mise en oeuvre in vitro selon les techniques connues de la culture lymphocytaire mixte. Ces techniques sont rappelées ci-après à propos de la culture du clone de lymphoblaste T.

Il est toutefois possible d'utiliser comme agents immunogènes des clones T sensibilisés obtenus par alloimmunisation in vivo, et l'invention a également pour objet un procédé, tel que défini ci-dessus, dans lequel, pour préparer le clone de lymphoblaste T, on recueille les cellules mononucléées infiltrant un greffon allogénique humain transplanté et ayant fait l'objet d'un rejet, et on isole selon les méthodes connues, parmi les cellules recueillies, un clone de lymphoblaste T de l'individu ayant rejeté le greffon. En outre, on cultive ledit clone, comme précédemment, par stimulations successives, in vitro, à l'aide de cellules allogéniques. Les cellules stimulantes peuvent être notamment des cellules du donneur de greffon et, comme précédemment, on pourra utiliser avantageusement, comme cellule stimulante, une lignée de cellules transformées cultivables in vitro, notamment une lignée obtenue par transformation de cellules B du donneur. Il convient de noter que l'on peut remplacer une telle lignée par des cellules allogéniques portant la spécificité HLA-D qui est reconnue par le clone considéré, ou par des lignées cellulaires obtenues à partir de telles cellules allogéniques.

On décrit ci-après des modes de réalisation particuliers du procédé de l'invention.

La première étape du procédé consiste à obtenir des cellules T. Pour séparer les lymphocytes T, sensibilisés ou non, des monocytes et des autres lymphocytes, on peut faire passer de façon connue les cellules sur des colonnes garnies de fibres de laine de nylon qui retiennent les cellules autres que les lymphocytes T.

Pour sélectionner les lymphocytes T activés, il convient d'abord d'obtenir des clones puis de les faire proliférer. Les clones peuvent être obtenus par dilution convenable d'une suspension de cellules T, selon les méthodes connues. La prolifération est mise en oeuvre dans des conditions classiques de la culture lymphocytaire mixte (ou FILM), par mise en présence des cellules T avec des cellules stimulantes allogéniques, de préférence des cellules B. On détermine préalablement la proportion de cellules T qui prolifèrent bien en culture mixte (cellules répondeuses). On effectue alors la culture lymphocytaire mixte proprement dite en utilisant par exemple 2 cellules stimulantes pour 1 cellule T répondeuse. Les cellules stimulantes sont inactivées, par exemple par irradiation, pour rendre la réaction unidirectionnelle.

Dans le cas où les cellules T de départ ont été obtenues à partir de cellules infiltrant un greffon rejeté, on isole, parmi ces cellules, les lymphoblastes, qui ont une densité caractéristique (centrifugation en gradient de densité) et qui n'adhèrent pas à la laine de nylon. En outre, les lymphoblastes portent à leur surface certains antigènes dits d'activation que l'on peut mettre en évidence par des tests connus (par exemple : immunofluorescence de surface). En particulier, ces cellules T activées portent un déterminant HLA DR absent sur les lymphocytes T circulants au repos.

D'une façon générale, pour repérer les clones de lymphoblastes, on recherche selon les méthodes connues, par exemple à l'aide d'anticorps monoclonaux commerciaux, la présence, sur les cellules du clone considéré, des marqueurs de différenciation et d'activation caractéristiques des lymphoblastes T, notamment les récepteurs pour l'Interleukine-2 (R-IL2) les antigènes HLA-DR, les marqueurs T4 ou T8.

Parmi les clones de lymphoblastes T, on sélectionne ceux qui ont une bonne capacité de prolifération, par exemple ceux qui donnent une prolifération importante au bout de 48 heures de RLM, cette prolifération se maintenant pendant 5 à 7 jours.

Parmi les clones doués d'une bonne capacité de prolifération, on sélectionne de préférence ceux qui sont cytotoxiques. La cytotoxicité est évaluée en culture lymphocytaire mixte selon les méthodes usuelles.

Pour obtenir un sérum antilymphocytaire selon l'invention, on immunise des animaux, tels que lapins ou chevaux, par administrations répétées, selon les méthodes connues, de cellules du clone T sélectionné. Par exemple, chez le lapin on effectue plusieurs administrations, par voie intraveineuse, ou sous-cutanée, avec ou sans adjuvant, de $10^6$ à $10^9$ cellules chaque fois, les administrations étant espacées d'au moins une semaine. Une semaine environ après la dernière immunisation, on recueille le sérum des animaux immunisés et isole selon les méthodes connues les anticorps sériques qui peuvent être purifiés de façon usuelle, par exemple par précipitation fractionnée à l'éthanol, au sulfate d'ammonium, au rivanol, au polyéthylèneglycol, etc..., ou encore par passage sur colonnes échangeuses d'ions. Les anticorps obtenus peuvent être soumis aux traitements classiques permettant leur administration par voie intraveineuse, par exemple les traitements de clivage enzymatique par la plasmine, la papaïne ou la pepsine.

L'invention a également pour objet un sérum antilymphocytaire obtenu par le procédé décrit ci-dessus.

L'invention a en outre pour objet un médicament constitué par ce sérum antilymphocytaire. Ce médicament est destiné à être administré à des humains devant subir ou ayant subi une transplantation d'organe (rein, coeur, moëlle osseuse, pancréas, foie), afin d'éviter le phénomène de rejet. Ce médicament est utilisé sous les formes d'administration et aux posologies usuelles pour les SAL. Par exemple, par voie intraveineuse, on peut administrer des doses de 1-2,5mg/kg par jour, du jour 0 au jour 13 inclus, ou pendant une période plus longue.

Les exemples suivants illustrent l'invention sans toutefois la limiter.

EXEMPLE 1 : Préparation de clones de lymphoblastes T

A. Isolement des lymphocytes.

Les lymphoblastes T ont été isolés à partir des cellules mononucléées infiltrant un greffon rénal allogénique humain transplanté et donnant lieu à un rejet chronique. Il s'agit donc de lymphocytes du receveur de rein, sensibilisés contre les cellules du greffon allogénique.

Bien entendu, on a vérifié, chez le donneur et le receveur, l'absence d'infections virales (hépatites, EBV, CMV, HIV).

Après découpage du cortex rénal, on fait passer les cellules obtenues sur un gradient de Ficoll/métrizoate de sodium (MSL Eurobio, Paris). On fait ensuite passer les cellules sur une colonne de laine de nylon prétraitée qui retient les monocytes, les macrophages et les lymphocytes B et la majorité des cellules T immatures.

B) Obtention d'une lignée de cellules stimulantes.

On sait que les lymphocytes T nécessitent pour leur culture in vitro un facteur de croissance glycoprotéique, l'interleukine 2 (IL-2) recombinante, ainsi qu'une mise en contact régulière avec l'antigène cellulaire contre lequel ils sont spécifiquement sensibilisés.

Afin de pouvoir disposer d'une source permanente de cellules stimulantes, et compte tenu du fait que les lymphoblastes T provenant du greffon rejeté peuvent être supposés, pour là plus grande partie, sensibilisés contre les antigènes d'histocompatibilité portés par les cellules du greffon, il est commode, afin de disposer d'une source permanente de cellules stimulantes, de faire subir à des cellules du donneur une transformation permettant de les cultiver in vitro .

Pour cela, on a transformé des lymphocytes B du donneur à l'aide du virus d'Epstein-Barr (EBV) de la façon suivante.

On prélève des cellules spléniques du donneur de rein selon une méthode classique ; voir par exemple B.B. MISHELL and S.M. SHIGI, Selected Methods in Cellular Immunology, FREEMAN, San Francisco, 1980. Les lymphocytes sont isolés après passage sur un gradient de Ficoll/métrizoate de sodium. Les cellules sont lavées, puis le culot cellulaire est remis en suspension dans un surnageant de culture, préalablement filtré sur filtre 0,45μm, d'une lignée cellulaire B 95.8 (ATCC n°CRL 1612) productrice du virus d'Epstein-Barr.

Après incubation pendant 2 heures à 37°C, les cellules sont centrifugées et le culot cellulaire est remis en suspension, à raison de $10^6$ cellules/ml, dans du milieu de culture RPMI 1640 à 10% de sérum de veau foetal décomplémenté, supplémenté avec 0,1μg/ml de cyclosporine A (Sandoz). Les cultures sont incubées à 37°C en atmosphère humide à 5% de $CO_2$. Une fois par semaine, on remplace le milieu de culture par du milieu de culture frais.

La lignée lymphoblastoïde B ainsi obtenue, appelée ci-après LLBD, est alors cultivée dans du milieu RPMI à 10% de sérum de veau foetal. On a vérifié que cette lignée n'exprime pas le virus EBV.

Une partie des cellules de cette lignée est prélevée et soumise à une irradiation de 50 grays. Les cellules irradiées, qui sont incapables de proliférer, sont utilisées comme cellules stimulantes spécifiques lors du clonage et de la culture à long terme du clone de lymphoblaste T.

On a préparé de la même façon une lignée de cellules B transformées, appelée ci-après LLBR, provenant du receveur du greffon.

C) Clonage de lymphoblastes T

La technique de clonage adoptée est la dilution limitante ; référence : H. WALDMAN, I. LEFKOVITS and J.QUINTANS, Limiting Dilution Analysis of Helper Cell Function, Immunology, 1975, 28: 1135.

On réalise une première dilution limitante afin d'établir la fréquence de prolifération des lymphocytes T répondeurs cultivés en présence de LLBD irradiée. Une fois cette fréquence déterminée, on ensemence des puits de culture à des concentrations cellulaires permettant l'obtention de colonies dont la probabilité de monoclonalité est supérieure à 97%.

Le clonage est réalisé en plaques de Terasaki de 60 puits (Nunc). Les puits ensemencés à une cellule répondeuse par puits en présence de 1000 cellules LLBD irradiées sont cultivées dans un milieu de culture standard : RPMI 1640 à 10% de plasma humain purifié agammaglobulinémique PHP : origine CRST, Lille, France), 0,94nM IL-2 recombinante (origine : ROUSSEL UCLAF). Après 12 jours de culture, on a observé la formation de 120 colonies. Compte tenu du pourcentage de puits vides observés (95%) la probabilité minimale de monoclonalité des colonies sélectionnées est supérieure à 97,5%.

Les 99 colonies sont repiquées en plaques à 96 puits de 200μl de capacité, et cultivées dans 150μl du milieu de culture précédent, en présence de 15,000 cellules stimulantes. Par la suite, ces clones ont été transférés en plaques à 24 puits de 2ml de capacité et cultivés dans 1ml du milieu de culture en présence de 500.000 cellules stimulantes, et enfin en flacon de culture de 50ml de capacité dans 10ml de milieu de culture en présence de $10^6$ cellules stimulantes.

A ce stade, on a obtenu 55 clones qui ont été congelés en attente d'utilisation.

D) Caractérisation phénotypique et fonctionnelle des clones

a) Marqueurs de différenciation

Il s'agit d'établir les sous-populations de cellules T auxquelles appartiennent les clones obtenus. Les marqueurs spécifiques de ces sous-populations sont des anticorps monoclonaux commerciaux (généralement des anticorps d'origine murine).

Le phénotypage est établi selon une technique d'immunofluorescence indirecte, en utilisant comme second anticorps des anticorps de chèvre anti-immunoglobuline de souris couplés à l'isothiocyanate de fluorescéine (Nordic, Norvège).

Tous les clones obtenus possèdent les marqueurs T3 et T11. L'expression des antigènes du complexe majeur d'histocompatibilité de classe 2 est variable. Il en va de même pour l'expression du récepteur pour l'interleukine-2 (R-IL2).

Environ 60% des clones possèdent le marqueur T4 et 40% le marqueur T8.

b) Antigènes d'activation

La recherche de ces antigènes a pour but de vérifier que les clones obtenus possèdent les marqueurs caractéristiques de l'activation (lymphoblastes).

L'expression des R-IL2 est évaluée avec des anticorps anti-TAC. Le taux de R-IL2 exprimé à la surface d'un clone dépend de la concentration des cellules stimulantes et du cycle de prolifération cellulaire.

Les clones expriment des taux variables de R-IL2 ; généralement les clones T4 expriment plus de R-IL2 en réponse à l'antigène que les clones T8 ; le marqueur Dr, absent des lymphocytes T au repos, est souvent présent, même en fin de cycle multiplicatif ..

c) Activité proliférative

Les clones, stimulés préalablement 4 jours avant l'expérience, sont ajustés à 50.000 cellules/ml. On répartit cette suspension sur des plaques à raison de 100μl par puits et on ajoute 100μl d'une suspension de cellules stimulantes irradiées à 500.000 cellules/ml. On évalue la prolifération de façon classique par la mesure de l'incorporation de thymidine tritiée au bout de 2 jours de test. Pour cela, on ajoute au bout de 2 jours 50μl de thymidine tritiée (1/100, TRK61, Amersham) et on laisse incuber 6 heures à 37°C. Les cellules sont récupérées sur filtres de fibre de verre (Skatron), et introduites dans du liquide à scintillation. La radioactivité est comptée dans un compteur bêta (LKB Wallach, USA).

On a observé qu'en général les clones T4 prolifèrent davantage que les clones T8.

d) Activité cytotoxique

Etant donné le rôle important joué par les lymphocytes T cytotoxiques dans le rejet des allogreffes, il est apparu souhaitable de sélectionner les clones ayant une activité cytotoxique.

L'activité cytotoxique des clones T a été établie selon un test de relargage du chrome radioactif. La technique utilisée est la suivante.

On met en suspension les cellules cibles (2 à $8.10^6$ cellules) dans 150μl d'une solution de bichromate de sodium radioactif ($^{51}$Cr) et on ajoute 50μl de sérum de veau foetal (SVF). Après une heure d'incubation à 37°C les cellules sont lavées avec 10ml de RPMI 1640, 5% SVF et ajustées à 30.000 cellules/ml, puis réparties dans des plaques à raison de 3.000 cellules par puits. Les cellules effectrices sont ajustées à 300.000 cellules/ml dans du RPMI 1640 SVF 5% puis sont réparties à raison de 100μl dans les puits. Après 4 heures d'incubation à 37°C, on prélève 100μl de surnageant et la radioactivité relarguée est comptée dans un compteur gamma.

RESULTATS :

Parmi les clones T4, qui prolifèrent bien, on a sélectionné 8 clones ayant une bonne activité cytotoxique. Dans l'exemple suivant, on va décrire plus particulièrement les propriétés d'un des clones étudiés, appelé 1E7.

EXEMPLE 2: Propriétés du clone 1E7

Les divers tests utilisés sont, sauf indication contraire, ceux mentionnés à l'exemple 1.

1) Phénotype de surface

a) Marqueurs de différenciation

Les résultats obtenus avec le clone 1E7 sont les suivants :
- 100% CD2 ; réactif : anticorps OKT11 (Ortho), anti-récepteur pour les globules rouges de mouton ;
- 100% CD3 ; anticorps : OKT3 (Ortho) ;
- 100% CD4 ; anticorps : OKT4 (Ortho) ou IOT4 (Immunotech) reconnaissant une sous-population T ;
- 0% CD8 ; anticorps OKT8 (Ortho) ou IOT8 (Immunotech) reconnaissant une sous-population T ;
- 100% WT31 (anticorps anti-chaînes alpha/bêta du récepteur T)
- 0% CD16 ; anticorps Leu 1 (Becton Dickinson), reconnaissant les cellules NK ;
- 0% OKM1 (Ortho), reconnaissant les monocytes/macrophages et une sous-population NK ;
- 100% W6/32 (anticorps anti HLA 1)
- expression variable du CD25 ; réactifs : anticorps 33B3.1 (Immunotech) ou anti-TAC (anti-R-IL2) ;
- expression variable des antigènes du complexe majeur d'histocompabilité (CMH) de classe 2.

b) Antigènes d'activation

L'expression des R-IL2, des récepteurs pour la transferrine (R-Tsf) et des différents produits des gènes CMH de classe 2 a été analysée en cytofluorimétrie au bout de différents temps après la stimulation antigénique.

Le clone 1E7 exprime intensément les différents antigènes d'activation testés (R-IL2, R-Tsf et antigènes CMH de classe 2). L'expression des R-IL2 et R-Tsf est strictement proportionnelle à l'intensité de la stimulation antigénique alors que celle des antigènes CMH de classe 2 semble optimale pour les rapports cellules stimulantes/cellules répondeuses (S/R) de 1/1.

Remarque : La lignée LLBD n'exprime pas de R-IL2, mais exprime des antigènes CMH de classe 2 et des R-Tsf.

### 2) Activité proliférative

L'activité proliférative du clone 1E7 est rigoureusement proportionnelle à la quantité de cellules stimulantes ajoutée en début de culture. Plus le rapport S/R est élevé, plus la prolifération est prolongée. On observe un pic de prolifération élevé deux jours après stimulation.

D'autre part, 6 jours après stimulation, son activité proliférative reste importante.

Enfin, le clone 1E7 semble reconnaître spécifiquement la LLBD puisque la LLBR ne stimule pas significativement sa prolifération.

### 3) Production de lymphokines

Le clone 1E7 ne produit pas de quantités d'IL2 détectables par un test de prolifération d'une lignée murine IL2-dépendante (CTL-2).

### 4) Activité cytotoxique

Le clone 1E7 est fortement cytotoxique vis-à-vis de la LLBD (121,6 LU 30/$10^6$ cellules) et rigoureusement non-réactif vis-à-vis de la LLBR et d'une lignée cancéreuse (lignée érythromyéloïde K562).

LU 30 signifie : unités lytiques donnant 30% de lyse spécifique.

Le fait que le clone 1E7 ne soit pas cytotoxique vis-à-vis de la LLBR montre qu'il ne reconnaît pas les déterminants du virus d'Epstein Barr utilisé pour la transformation. Le fait qu'il ne soit pas cytotoxique vis-à-vis de la lignée K562 montre qu'il est dépourvu d'activité Natural Killer (NK).

### 5) Répertoire de reconnaissance

L'activité proliférative et cytotoxique du clone 1E7 a été testé vis-à-vis d'un ensemble de lignées allogéniques partageant des spécificités HLA avec les cellules du donneur de rein. De plus, on a étudié l'effet de l'addition d'anticorps monoclonaux dirigés contre les produits de différents gènes CMH de classe 2.

La technique utilisée est la suivante. 7 jours après la dernière stimulation, les cellules sont passées sur gradient de Ficoll, puis isolées et lavées. Les culots cellulaires sont alors remis en suspension dans des milieux de culture supplémentés en IL2 recombinante et ajustés à 100.000 cellules/ml. Les cellules stimulantes sont irradiées et ajustées à 500.000 cellules/ml. On ajoute 0,1 ml de suspension de cellules répondeuses (1E7) à 0,1ml de cellules stimulantes. Les cellules sont cultivées pendant 3 jours. On ajoute de la thymidine tritiée 18 heures avant la récupération des cellules sur filtre.

Pour le bloquage de la cytotoxicité, on a utilisé les anticorps monoclonaux suivants : W6/32 et 41H.611 (anti-HLA I), BT2/9 (anti-DP/DR monomorphe) GSP4.1 et BM50 (anti-DR), Leu10 et 1A3 (anti-DQ) et B7.21 (anti-DP).

### RESULTATS

Le clone 1E7 semble reconnaître une spécificité DRw8 car il reconnaît les cellules allogéniques porteuses de cette spécificité et son activité cytotoxique proliférative vis-à-vis de la LLBD est bloquée par des anticorps dirigés contre des épitopes DR monomorphes (GSP4.1), mais pas par des anticorps reconnaissant des antigènes DQ, DP ou HLA I.

Remarque : il est possible de stimuler le clone 1E7 en remplaçant la lignée LLBD par des cellules portant la spécificité DRw8 ou par une lignée hybride obtenue à partir de telles cellules.

### Réarrangements des gènes du récepteur T

Le recepteur des lymphocytes T est un hétérodymère constitué de chaînes alpha et bêta codées par un ensemble de gènes dont l'organisation génomique est similaire à celle des gènes des immunoglobulines. Chaque gène alpha et bêta est le produit de gènes variables (V) et d'autres gènes (gènes de jonction J, gènes de diversité D, et gènes constants C) qui sont génomiquement réarrangés durant la maturation du lymphocyte T. Un troisième gène, dénommé gamma, est également spécifiquement réarrangé chez les lymphocytes T. Le nombre de produits réarrangés alpha, bêta et gamma différents est très élevé et chaque combinaison alpha, bêta, gamma est spécifique d'un clone lymphocytaire T donné. De plus, un lymphocyte T ne pouvant réarranger que les deux gènes alpha, bêta ou gamma sur ses deux chromosomes, l'analyse des réarrangements des gènes constitue un test direct de monoclonalité. En effet, la détection de plus de deux réarrangements alpha, bêta ou gamma chez une colonie lymphocytaire T signifie obligatoirement que ces cellules sont polyclonales. Les remaniements génomiques décrits ci-dessus induisant une modification de la carte de restriction des gènes récepteurs T, il est possible de détecter ces réarrangements après digestion de l'ADN avec des enzymes de restriction appropriés, migration sur gel d'agarose et transfert sur filtre (technique de Southern), puis hybridation avec une sonde radiomarquée reconnaissant les gènes alpha, bêta ou gamma du récepteur T. Sur la lignée antologue LLBR, qui ne réarrange pas ses gènes du récepteur T, la sonde révèlera des bandes dites germinales qui auront une taille donnée. Un réarrangement induisant une modification de la position des sites de restriction par rapport à la sonde, on peut ainsi détecter chez les lymphocytes T matures des bandes dites réarrangées qui migreront à des positions différentes de celles de la bande germinale, ou des bandes germinales, dans le gel d'agarose.

Les résultats obtenus sont parfaitement compatibles avec une monoclonalité du lymphocyte 1E7. Le clone 1E7 est un clone ayant réarrangé les gènes TRG et TCR sur ses deux chromosomes (son statut étant 2 réarrangements V/J/C 2 et réarrangement V/D/J/C 2).

EXEMPLE 3: Préparation de sérum antilymphocytaire

On immunise des lapins par administration de $3.10^8$ cellules 1E7 par injection. On procède à 3 injections par voie intraveineuse aux jours 0, 14 et 21. En outre, au jour 5, on administre par voie intraveineuse 10 doses de BCG à $10^7$-$10^8$ germes/10 doses. Le sérum est collecté au jour 28, par saignée. On recueille environ 100 ml de sérum par lapin.

Les anticorps sont purifiés de la façon suivante.

a) Chromatographie d'échange d'ions sur DEAE cellulose (diéthylaminoéthyl cellulose)

On chromatographie sur DEAE cellulose Whatman, et on élue avec un tampon phosphate disodique 1,5g/l, pH 8.

b) Précipitation au sulfate de sodium

La solution de gammaglobuline obtenue est purifiée par double précipitation avec du sulfate de sodium à 180 g/l, puis 170g/l, pH 7. Le précipité est redissous dans une solution de glycocolle 0,3 M, pH 7, pour obtenir un volume égal au volume de départ.

c) Adsorption sur globules rouges humains frais

La solution est hémadsorbée deux fois sur des culots de globules rouges humains (volume de culot pour chaque adsorption sensiblement égal au volume de sérum brut), afin d'abaisser le taux d'hémagglutinines.

La solution est à nouveau précipitée par le sulfate de sodium afin d'éliminer l'hémoglobine.

Le précipité est dissous dans un tampon glycocolle 0,3 M, diafiltré contre une solution finale de glycocolle 10g/l, NaCl 2 g/l, mannitol 10g/l. On ajoute les protéines à 5g/l, puis on lyophilise. Le sérum antilymphocytaire obtenu est appelé ci-après GAB (gammablobulines anti-blastes).

EXEMPLE 4: Utilisation du sérum antilymphocytaire obtenu dans la prophylaxie du rejet aigu des transplantations rénales.

Cette étude a porté sur 12 patients ayant reçu une greffe de rein. Tous ont reçu pendant 14 jours, à compter du jour de la transplantation, une dose de 1,5-2,5 mg/kg et par jour (exprimée en immunoglobulines) de sérum antilymphocytaire GAB provenant d'un même lot de fabrication (voie intraveineuse).

Les patients subissaient par ailleurs un traitement temporaire avec des médicaments immunosuppresseurs associés : corticoïdes et azathioprine. D'autres médicaments étaient systématiquement prescrits dans cette période initiale : pénicilline G et couverture antibiotique post-opératoire.

Dès le 14ème jour suivant la greffe, la cyclosporine A est introduite à la dose de 8 mg/kg/jour. Cette posologie initiale est ensuite modifiée en fonction du dosage radioimmunologique de la cyclosporine A sur sang total. La cyclosporine en monothérapie constitue ensuite le traitement au long cours de la greffe.

La compatibilité entre donneur et receveur dans le système HLA, évaluée par sérologie, était : supérieure ou égale à 3 pour 7 patients ; 1 pour 4 patients ; nulle pour 1 patient.

RESULTATS :

REJETS

On n'a noté aucun rejet aigu pendant la période de traitement avec le GAB. Des épisodes de rejet sont survenus de façon retardée, après l'arrêt du traitement :
- au 31ème jour de la greffe chez un patient (pour lequel il s'agissait d'une deuxième greffe) ;
- au 36ème jour de la greffe chez un patient (patient hyperimmunisé pour lequel il s'agissait d'une 2ème greffe).

Par ailleurs, un rejet est intervenu aux 22ème et 60ème jours de la greffe chez un patient, après interruption volontaire par le patient, à deux reprises, du traitement par la cyclosporine A, et au 80ème jour chez un autre patient.

Dans tous les cas, la crise de rejet a été reversible par administration de sérum antilymphocytaire.

FONCTION RENALE

Le délai de démarrage de la fonction rénale après la transplantation, qui est en moyenne de 6 jours, est strictement identique à celui observé avec les SAL classiques.

TOLERANCE

Quelques effets secondaires mineurs ont été observés :
- une éruption cutanée au 3ème jour qui s'est résorbée spontanément en 48 heures, et qui est probablement imputable à la' pénicilline ;
- une légère fièvre (température corporelle : 37,5-38°C) chez trois patients.

La seule réaction d' intolérance a été une maladie sérique aiguë d'intensité modérée, au 14ème jour de traitement, chez un patient.

Le nombre de plaquettes n'a pas été abaissé.

TAUX DE LYMPHOCYTES T CIRCULANTS

Ce taux a été déterminé par cytofluorimétrie, en utilisant des anticorps monoclonaux anti-CD2, -CD3, -CD4 et -CD8.

Les populations lymphocytaires CD2$^+$ et CD3$^+$ et les sous-populations CD4$^+$ et CD8$^+$ ont évolué de façon similaire. Pour toutes les populations lymphocytaires, on a observé un effondrement au 2ème jour, et une remontée progressive du jour 14 au jour 30, jusqu'à des valeurs toujours inférieures aux valeurs antérieures à la greffe.

EXEMPLE 5 : ETUDE DES EFFETS CYTOTOXIQUES DU GAB (Gammaglobulines anti-blastes obtenues selon l'invention)

On a préparé deux lots différents de GAB par immunisation de lapins, selon la technique décrite à l'exemple 3, à l'aide de cellules 1E7 comparables (même nombre de passages prolifératifs). Un des lots (lot n° 1) a été préparé à partir de cellules 1E7 fraîches. L'autre lot (lot n°2) a été préparé à partir de cellules 1E7 congelées dans l'azote liquide puis décongelées.

Activité cytotoxique

Les cellules cibles sont placées dans 1 µl de milieu de culture, dans des puits de plaques de Terasaki, le milieu de culture étant constitué de RPMI à 10% de SVF. On ajoute ensuite 5µl de la dilution de GAB. Ces dilution sont effectuées dans du tampon PBS à 0,1% de BSA (Sérum albumine bovine). Après une demi-heure d'incubation à température ambiante, on ajoute 5µl de complément de lapin et les plaques sont laissées pendant 3/4 d'heure à température ambiante. Les résultats sont exprimés en pourcentages de cellules mortes (lecture au microscope).

Les cellules testées ont été les suivantes :

| | |
|---|---|
| PBL...: | Lymphocytes du sang périphérique humain |
| Blastes : | Cellules obtenues au bout de 4 jours après stimulation des PBL par la phytohémagglutinine (PHA) |
| U937....: | Lignée promonocytaire |
| Dabin...: | Lignée cellulaire B |
| Jurkat..: | Lignée T humaine |
| YT .....: | Lignée humaine de type NK |
| 1E7.....: | Clone décrit aux exemples 1 et 2 ci-dessus |
| 2B11....: | Clone T4 non cytotoxique autologue de 1E7 |
| 1B5.....: | Clone T8 cytotoxique |

Les résultats sont résumés dans le tableau I suivant.

On constate que les lots GAB 1 et GAB 2, on un effet cytotoxique analogue vis à vis des cellules testées, même si de petites variations sont observées dans la sensibilité des cellules les unes par rapport aux autres.

## EP 0 335 804 A1

### TABLEAU I

### CYTOTOXICITE. POURCENTAGE DE CELLULUES MORTES

| CEL-LULES | GAB Lot n° | MI | C' | DILUTION DU GAB | | | | | | | |
|---|---|---|---|---|---|---|---|---|---|---|---|
| | | | | 40 | 80 | 160 | 320 | 640 | 1280 | 2560 | 5120 |
| PBL | 1 | 0 | 0 | 100 | 100 | 100 | 100 | 75 | 25 | 0 | 0 |
| | 2 | 0 | 0 | 100 | 100 | 100 | 100 | 75 | 25 | 0 | 0 |
| BLAST-ES | 1 | 0 | 0 | 100 | 100 | 100 | 100 | 75 | 50 | 0 | 0 |
| | 2 | 0 | 0 | 100 | 100 | 100 | 100 | 75 | 50 | 0 | 0 |
| U937 | 1 | 0 | 0 | 100 | 100 | 100 | 100 | 25 | 0 | 0 | 0 |
| | 2 | 0 | 0 | 100 | 100 | 100 | 100 | 50 | 0 | 0 | 0 |
| DABIN | 1 | 0 | 0 | 100 | 100 | 100 | 100 | 50 | 0 | 0 | 0 |
| | 2 | 0 | 0 | 100 | 100 | 100 | 100 | 50 | 0 | 0 | 0 |
| JURKAT | 1 | 0 | 0 | 100 | 100 | 100 | 100 | 100 | 75 | 50 | 0 |
| | 2 | 0 | 0 | 100 | 100 | 100 | 100 | 100 | 75 | 50 | 0 |
| YT | 1 | 0 | 0 | 100 | 100 | 100 | 100 | 50 | 25 | 25 | 0 |
| | 2 | 0 | 0 | 100 | 100 | 100 | 100 | 50 | 25 | 25 | 0 |
| 1E7 | 1 | 0 | 0 | 100 | 100 | 100 | 100 | 100 | 25 | 0 | 0 |
| | 2 | 0 | 0 | 100 | 100 | 100 | 100 | 100 | 25 | 0 | 0 |
| 2B11 | 1 | 0 | 0 | 100 | 100 | 100 | 100 | 50 | 0 | 0 | 0 |
| | 2 | 0 | 0 | 100 | 100 | 100 | 100 | 50 | 0 | 0 | 0 |
| 1B5 | 1 | 0 | 0 | 100 | 100 | 100 | 100 | 25 | 0 | 0 | 0 |
| | 2 | 0 | 0 | 100 | 100 | 100 | 100 | 50 | 0 | 0 | 0 |

MI : milieu de culture seul

C' : complément seul

## Revendications

1. Procédé de préparation d'un sérum antilymphocytaire, dans lequel on immunise des animaux vertébrés à l'aide d'un immunogène constitué par des cellules lymphoïdes humaines, et l'on recueille au bout d'un temps suffisant les anticorps sériques des animaux immunisés, caractérisé par le fait que l'on prépare un clone de lymphoblaste T humain sensibilisé contre des cellules allogéniques, et que l'on utilise des cellules de ce clone comme immunogène.

2. Procédé selon la revendication 1, caractérisé par le fait que, pour préparer ledit clone, on sensibilise des lymphocytes T humains à l'aide de cellules allogéniques, puis l'on isole, selon les méthodes connues, un clone de lymphoblaste allosensibilisé.

3. Procédé selon la revendication 2, caractérisé par le fait qu'en outre on cultive ledit clone par stimulations successives à l'aide de cellules allogéniques.

4. Procédé selon la revendication 3, caractérisé par le fait que les cellules stimulantes sont des cellules transformées cultivables in vitro.

5. Procédé selon la revendication 4, caractérisé par le fait que lesdites cellules stimulantes proviennent du même individu que celles ayant servi à réaliser l'allosensibilisation.

6. Procédé selon la revendication 1, caractérisé par le fait que, pour préparer ledit clone, on recueille les cellules mononucléées infiltrant un greffon allogénique humain transplanté et ayant fait l'objet d'un rejet et on isole, selon les méthodes connues, parmi les cellules recueillies, un clone de lymphoblaste T de l'individu ayant rejeté le greffon.

7. Procédé selon la revendication 6, caractérisé par le fait qu'en outre on cultive ledit clone par stimulations successives à l'aide de cellules allogéniques.

8. Procédé selon la revendication 7, caractérisé par le fait que les cellules stimulantes sont des cellules transformées cultivables in vitro.

9. Procédé selon la revendication 7 ou 8, caractérisé par le fait que les cellules stimulantes sont des cellules du donneur de greffon ou des cellules obtenues par transformation des cellules dudit donneur.

10. Sérum antilymphocytaire, caractérisé par le fait qu'il est obtenu selon le procédé de l'une quelconque des revendications précédentes.

9

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 216 998 (CHOAY S.A.) <br> * Page 3, lignes 28-31; page 4, lignes 39-40; page 5, lignes 1-22 * <br> --- | 1-10 | A 61 K 39/395// <br> C 12 N 5/00 |
| Y | FR-A-2 153 193 (AGENCE NATIONALE DE VALORISATION DE LA RECHERCHE) <br> * Page 7, lignes 36-40; page 8, lignes 1-2; page 16, revendication 1 * <br> --- | 1-10 | |
| Y | WO-A-8 402 848 (CENTOCOR INC.) <br> * Page 5, lignes 9-27; page 4, lignes 12-17; page 7, lignes 9-19; page 12, lignes 5-15 * <br> --- | 1-10 | |
| A | ANNALS OF SURGERY, vol. 170, no. 4, 1969, pages 617-632, J.B. Lippincott Co., Philadelphia, US; J.S. NAJARIAN et al.: "Anti-serum to cultured human lymphoblasts: Preparation, purification and immunosuppressive properties in man" <br> * En entier * <br> ----- | 1-10 | |

**DOMAINES TECHNIQUES RECHERCHES (Int. Cl.4)**

A 61 K

Le présent rapport a été établi pour toutes les revendications

| Lieu de la recherche | Date d'achèvement de la recherche | Examinateur |
|---|---|---|
| LA HAYE | 28-06-1989 | FERNANDEZ Y BRANAS F.J. |